# EUROPÄISCHE PATENTSCHRIFT

(11) **EP 0 920 304 B1**
(45) Veröffentlichungstag und Bekanntmachung des Hinweises auf die Patenterteilung: **08.08.2001**
(21) Anmeldenummer: 98922771.5
(22) Anmeldetag: 23.04.1998
(51) Int. Cl.: A61K 9/16

(54) **VERFAHREN ZUR HERSTELLUNG EINES GRANULATES FÜR DIE HÄMODIALYSE**
METHOD FOR THE PRODUCTION OF A GRANULATE FOR HEMODIALYSIS
PROCEDE DE FABRICATION D'UN GRANULAT POUR HEMODIALYSE

(30) Priorität: 24.04.1997 DE 19717362
(43) Veröffentlichungstag der Anmeldung: 09.06.1999
(73) Patentinhaber: Fresenius Medical Care Deutschland GmbH, 61350 Bad Homburg v.d.H. (DE)
(72) Erfinder: BACKHAUS, Wendelin, D-35789 Weilmünster (DE); EL-AYARI, Hamadi, D-60316 Frankfurt (DE); HILGERS, Peter, D-35580 Wetzlar (DE); LIEDY, Werner, D-67126 Hochdorf-Assenheim (DE); MANKE, Joachim, D-35792 Löhnberg (DE)
(74) Vertreter: Laufhütte, Dieter, Dr.-Ing.
(86) Internationale Anmeldenummer: EP9802425
(87) Internationale Veröffentlichungsnummer: WO9847488

(56) Entgegenhaltungen:
- EP-A- 0 602 921
- DATABASE WPI Section Ch, Week 9420 Derwent Publications Ltd., London, GB; Class B,Page 05, AN 94-163157 XP002076233 & JP 06 105 906 A (NIKKISO CO LTD) , 19. April 1994

## Beschreibung

Die Erfindung betrifft ein Verfahren zur Herstellung eines Granulats zur Bereitstellung einer bicarbonathaltigen Dialysierflüssigkeit für die Hämodialyse notwendigen Elektrolyten nach dem Oberbegriff des Anspruchs 1.

Die Dialyse als medizinisches Behandlungsverfahren bei Patienten mit gestörter oder verlorengegangener Nierenfunktion einschließlich seiner vielfältigen technischen Aspekte ist in der Literatur umfangreich beschrieben.

Bei der Hämodialyse werden für eine Behandlung etwa 120-180 Liter Dialysierflüssigkeit benötigt. Dabei handelt es sich um eine wäßrige Lösung von typischerweise folgender Zusammensetzung:

| Komponente | Konzentrationsbereich | typische Konzentration |
|---|---|---|
| Na⁺ | 125-150 mmol/l | 138 mmol/l |
| K⁺ | 0-5 mmol/l | 2 mmol/l |
| Ca⁺⁺ | 0,3-2,5 mmol/l | 1,5 mmol/l |
| Mg⁺⁺ | 0-1,5 mmol/l | 0,5 mmol/l |
| Cl⁻ | 92-120 mmol/l | 110 mmol/l |
| HCO₃⁻ | 30-40 mmol/l | 32 mmol/l |
| Acetat | 1,5-4 mmol/l | 3 mmol/l |
| Glukose | 0-3 g/l | 1 g/l |

Die Dialysierflüssigkeit wird in einem Dialysegerät durch Mischung von Elektrolytkonzentraten mit Reinstwasser hergestellt. Die Zusammensetzung muß dabei den physiologischen Erfordernissen entsprechen, da die Dialysierflüssigkeit über eine semipermeable Membran mit dem Blut des Patienten in Kontakt kommt.

Die Problematik einer geeigneten, physiologischen Zusammensetzung der Dialysierflüssigkeit sind z.B. ausführlich in dem Buch "Replacement of Renal Function by Dialysis" (Herausgeber: W. Drukker, F. M. Parsons, J. F. Maher-, Verlag Martinus Mijhoff Medical Division Den Haag) im Hauptabschnitt "the Composition of Dialysis fluid" (Verf.: F. M. Parsons, A. M. Davison) dargelegt.

Es ist bekannt und wird auch aus dieser Veröffentlichung ersichtlich, daß die Zusammensetzung der Dialysierflüssigkeit an die individuellen Bedürfnisse des einzelnen Patienten angepaßt werden sollte.

Da 120-180 Liter Dialysierflüssigkeit für eine vier- bis sechsstündige Behandlung erforderlich sind, ist es zweckmäßig, die Dialysierflüssigkeit am Ort der Anwendung zuzubereiten.

Es wurden Dialysegeräte entwickelt, bei denen die Dialysierflüssigkeit mittels automatischer Dosiereinrichtungen kontinuierlich oder in kleinen Teilmengen hergestellt wird. Dies geschieht gewöhnlich in der Weise, daß ein vorgefertigtes flüssiges Konzentrat in einem bestimmten volumetrischen Verhältnis (standardgemäß eine Volumeneinheit Konzentrat auf 35 Teile der fertigen Lösung) mit Reinstwasser verdünnt wird. Für dieses Verfahren stehen Flüssigkonzentrate in vielfältigen Varianten der Zusammensetzung zur Verfügung.

Die Applikation der Dialysekonzentrate an den Geräten erfolgt auf unterschiedliche Art und Weise: Eine übliche Technik ist die der sogenannten Zentralversorgung. Hier wird eine größere Anzahl von Dialysegeräten eines Zentrums, von einer zentralen Stelle aus über ein Leitungssystem mit Konzentrat versorgt.

Ein schwerwiegender Nachteil der zentralen Konzentratversorgung ist jedoch, daß eine individuelle Anpassung der Zusammensetzung an die Bedürfnisse des einzelnen Dialysepatienten nicht möglich ist, da über ein Konzentratversorgungsnetz jeweils nur eine Konzentratsorte verteilt werden kann.

Am weitesten verbreitet ist die Verwendung von Konzentratkanistern am einzelnen Dialysegerät. Das Gerät ist dann an eine Versorgungsleitung angeschlossen, über die aufbereitetes und in seiner Qualität kontrolliertes Reinstwasser zugeführt wird. Das Konzentrat wird in handelsüblichen Behältern (z.B. 6 oder 10 Liter) dem Dialysegerät beigestellt und über eine in den Behälter geführte Einrichtung von dem Gerät aufgenommen.

Ein großer Vorteil dieses Verfahrens ist, daß für die Behandlung jedes einzelnen Patienten ein besonders geeignetes Konzentrat ausgewählt werden kann. Eine solche unter dem Begriff "Individualisierung" bekannte Anpassung ist allgemein als wichtig anerkannt.

Die Verwendung von Konzentraten in Kanistern bedeutet für das auf Dialysestationen tätige Personal eine erhebliche Belastung, da die Konzentratbehälter bis zu 10 Kg wiegen. Die Lagerhaltung unterschiedlicher Konzentrate in derart großen Behältern erfordert erheblichen Aufwand. Für eine Dialysestation mit 20 Behandlungsplätzen liegt die typische Vorratsmenge für eine Woche in der Größenordnung von 200-300 Behältern. Reste von nicht aufgebrauchtem Konzentrat, die nach der Behandlung im Behälter verbleiben, sollen aus verschiedenen Gründen nicht weiterverwendet werden. Die Beseitigung dieser Reste, die durchaus bis zu 30 Prozent der eigentlichen Bedarfsmenge betragen können, stellt eine nicht unbeträchtliche Umweltbelastung dar. Die gebrauchten leeren Behälter, bei denen es sich gewöhnlich um Kanister aus hochwertigen Thermoplasten handelt, sind nach bisherigem Stand der Technik und unter Berücksichtigung wirtschaftlicher und organisatorischer Kriterien für den gleichen Zweck nicht wieder verwendbar. Ihre Rückführung in den Rohstoffkreislauf durch Recycling ist jedoch ebenfalls kein sehr wirtschaftliches Verfahren.

Hinzu kommt, daß bikarbonathaltige Dialysierflüssigkeit nicht aus nur einem einzigen Lösungskonzentrat herstellbar ist. Konzentrate die Mg⁺⁺ und/oder Ca⁺⁺ enthalten, sind nur ohne Bikarbonat stabil, da es zusammen mit Bikarbonat zu Karbonat-Ausfällungen käme.

Die Bikarbonatdialyse erfordert daher die Verwendung von mindestens zwei getrennten Konzentraten, nämlich eines Bikarbonat Konzentrats, gewöhnlich in der Form einer reinen Natriumbikarbonat-Lösung, und eines Konzentrats der anderen Lösungsbestandteile, wobei für das letztere die Bezeichnung "saures Bikarbonat-Hämodialyse-Konzentrat" üblich ist. Die für die Bikarbonat-Dialyse vorgesehenen Hämodialysegeräte müssen mit zwei getrennten Proportionierungseinrichtungen für diese beiden Konzentrate ausgerüstet sein.

Die Gefahr einer Ausfällung von Karbonaten entsteht bei diesem Verfahren erst nach dem Vermischen der Komponenten. Sie ist bei der Hämodialyse jedoch nur von geringer Bedeutung, da die Verweildauer der fertigen Dialysierflüssigkeit im Leitungssystem des Dialysegerätes einschließlich des Dialysators nur in der Größenordnung von etwa einer Minute liegt.

Ein weiteres Problem der Bikarbonatdialyse besteht darin, daß die handelsübliche Bikarbonat-Lösung nicht autosteril ist. Das Bikarbonat-Konzentrat muß daher mit erhöhter Sorgfalt steril produziert und bis zum Verbrauch in geeigneten Behältern steril gelagert werden.

Die oben beschriebenen flüssigen Konzentrate enthalten bis zu 80% Wasser und sind daher aus logistischer Sicht sehr unvorteilhaft.

Neben den flüssigen Konzentraten gibt es die Möglichkeit, Konzentrate aus den Rohsalzen vor Ort herzustellen. Die Salze müssen dann vor der Behandlung in Reinstwasser aufgelöst werden. Die Herstellung erfordert große Sorgfalt und ist sehr zeitaufwendig.

Eine Alternative bilden Granulate, die ausschließlich aus den für die Dialyse erforderlichen Substanzen bestehen. So sind bereits Bikarbonatgranulate als erstes, basisches Konzentrat bekannt, wobei der Rest der Gesamtlösungsbestandteile, der aus den Elektrolyten (Mg²⁺, Ca²⁺, K⁺, Na⁺) und einer physiologisch verträglichen Säure besteht, die in einem zweiten Konzentrat zusammengefaßt sind. Es ist auch schon bekannt, den Kochsalzanteil aus dem sauren Elektrolytanteil herauszunehmen und zur individuellen Patientenbehandlung als ein drittes Konzentrat zu verabreichen. Dies ist in der EP 0613688 bereits beschrieben.

Beispielsweise aus der EP 0 602 014 A1 ist es auch bereits bekannt, die Elektrolytanteile in Granulatform zur Verfügung zu stellen. Hier wurde bereits beschrieben, daß die gewünschte Essigsäuremenge diesem Granulat der Elektrolyte zugegeben wird. Allerdings besteht bei dem Stand der Technik die Gefahr, daß die dort hergestellten Granulate durch die Aufnahme der Essigsäure ihre Rieselfähigkeit verlieren.

Aufgabe der Erfindung ist es, ein Verfahren zur Herstellung eines Granulates der zur Bereitstellung einer bikarbonathaltigen Dialysierflüssigkeit für die Hämodiatyse notwendigen Elektrolyten zusammen mit der gewünschten Menge Essigsäure in einer Form an die Hand zu geben, in der das Granulat ein einfach zu verarbeitendes rieselfähiges Schüttgut darstellt.

Diese Aufgabe wird durch ein Verfahren zur Herstellung des gewünschten Granulates nach der Merkmalskombination des Anspruchs 1 gelöst. Bei dem erfindungsgemäßen Verfahren werden die Salze zunächst in einer Korngröße von kleiner als 0,5 mm eingesetzt und anschließend in trockener Gasatmosphäre auf eine Korngröße von 1-10 mm, vorteilhaft von ca. 2 mm, granuliert, wobei ein Porenvolumen im Granulat von größer als 25 Vol.-% erzeugt wird. Anschließend wird die Essigsäure im Vakuum auf das Granulat aufgesprüht. Durch diese kombinierten Verfahrensschritte wird ein Porenvolumen von mehr als 25% erzeugt, wobei es möglich ist, die gesamte benötigte Menge an Essigsäure in den Poren dieses Granulats aufzunehmen. Bei der erfindungsgemäßen Lösung wird zudem das Azetat, das bei den Konzentraten nach dem Stand der Technik enthalten ist, nicht mitgranuliert.

Bevorzugt wird ein Granulat hergestellt, das ein Porenvolumen zwischen 37 Vol.% und 60 Vol.% aufweist. Bei einem derartig hohen Porenvolumen ist es ohne weiteres möglich zwischen 15 und 35 Gew.-% Essigsäure pro Gesamtelektrolytmenge, idealerweise zwischen 18 und 20 Gew.-%.

Bevorzugte Ausgestaltungen des erfindungsgemäßen Verfahrens ergeben sich aus den sich an den Hauptanspruch anschließenden Unteransprüchen.

Obwohl unterschiedliche Granulierverfahren angewandt werden können, hat sich als besonders vorteilhaft der Einsatz von Wirbelbetten zur Herstellung der Granulate erwiesen. Hier bieten sich insbesondere die folgenden beiden bekannten Techniken an:
- Granulieren von zumeist als feinem Pulver vorgelegten Feststoffen zu größeren Granulaten unter Aufdüsen von Lösungsmittel und Abtrocknen dieses Lösungsmittels im Wirbelbett.
- Herstellung von Granulaten ausgehend von einer Vorlage aus festen Partikeln unter Aufdüsen der in einem Lösungsmittel aufgelösten Feststoffe und Abtrocknen dieses Lösungsmittels im Wirbelbett.

Das bevorzugte Lösungsmittel ist erfindungsgemäß polar, besonders bevorzugt Wasser.

Beide Verfahren sind geeignet, Granulate mit der gewünschten Porenstruktur herzustellen, die ein Aufnehmen der Essigsäure in ihrem Porenvolumen ohne weiteres ermöglicht.

Besonders vorteilhaft ist die Kombination der beiden Verfahrensvarianten, wobei sich beide Varianten in einem Wirbelbett durchfahren lassen. Durch Einstellen der Anteile verschiedener Komponenten der "Vorlage" und der "in Lösung aufgedüsten Feststoffe" lassen sich die Eigenschaften des Porensystems unter Berücksichtigung der Rezeptur optimieren.

Es hat sich insbesondere als vorteilhaft erwiesen, diejenigen Komponenten in gelöster Form aufzusprühen, die sich in ihrer Partikelgrößenverteilung deutlich von den anderen Ausgangsstoffen unterscheiden. Hierdurch werden Entmischungserscheinungen und damit einhergehende Inhomogenitäten des Granulats vermieden. Die Durchtrocknung (End- bzw. Resttrocknung) kann zur mechanischen Schonung der Granulate auch im Wirbelbett oder in einem Schachttrockner oder in einem anderen Trockner erfolgen.

Durch die erfindungsgemäße Verfahrensführung kann ein Porensystem mit besonderen Eigenschaften im Granulat geschaffen werden. Hier wird zunächst Einfluß auf die Porenradienverteilung genommen, wobei diese vom Radius der eingesetzten Partikel abhängen. Somit können gezielt feine Poren im Inneren geschaffen werden und grobe Poren im äußeren Bereich. Die feinen Poren im inneren Bereich dienen einem guten Einsaugen der Essigsäure aufgrund der wirkenden Kapillarkräfte, während die groben Poren im äußeren Bereich zur guten Rieseifähigkeit der Granulate führen. Zusätzlich können die Eigenschaften der inneren Oberflächen gezielt beeinflußt werden, wobei die Oberflächenspannung und/oder Grenzflächenspannung zwischen Feststoffoberfläche und der flüssigen Essigsäure dadurch beeinflußbar ist, daß eine oder mehrere vorbestimmte Komponenten über die in Lösung aufgedüsten Feststoffe auf die innere Oberfläche zur Verbesserung des Benetzungsverhaltens der Essigsäure aufgebracht werden können.

Das Aufbringen der Essigsäure, das vorteilhafterweise unter Vakuum erfolgt, kann beispielsweise in einem mechanisch schonenden Mischer oder aber auch nach dem Einfüllen der Granulate in einen Behälter aufgedüst werden.

Ein erfindungsgemäßes Granulat ergibt sich aus dem Anspruch 14.

Weitere Einzelheiten und Vorteile der Erfindung werden anhand von Beispielen, die bevorzugte Ausführungen des erfindungsgemäßen Verfahrens beschreiben, im folgenden dargestellt.

Die typische Zusammensetzung des durch das erfindungsgemäße Verfahren hergestellten Granulats ergibt sich wie folgt:

| | |
|---|---|
| CaCl₂ | 50 Gew.-% |
| KCI | 15 Gew.-% bis 35 Gew.-% |
| MgCl₂ | 15 Gew.-% bis 35 Gew. % |
| Essigsäure | 35 Gew.-% Massenbeladung bezogen auf die Gesamtsalzmasse. |

| | |
|---|---|
| Optional | |
| Glucose | 0 bis doppelter Gewichtsanteil der gesamten Salzmasse |

Die beispielhafte Verfahrensabfolge ergibt sich wie folgt:
1. Einwiegen der Komponenten Salze, zu versprühendes Wasser und gegebenenfalls Glucose;
2. Aufheizen des Sprühwirbelbettgranulators auf eine Betriebstemperatur von ca. 80°C;
3. Mahlen der drei Salze (MgCl₂, CaCl₂ und KCI) auf eine Komgröße von ≤ 0,5 mm. Die Salze sollen vorteilhaft einzeln bzw. getrennt voneinander zerkleinert werden, da es ansonsten zur Karnalitbildung kommen kann;
4. Einbringen der drei Salze in den Sprühwirbelbettgranulator;
5. Regulierung des Heizgasvolumenstromes, so daß sich ein einheitliches Wirbelbett ausbildet;
6. Aufheizen des Wirbelbettes auf Betriebstemperatur und gleichzeitiges Homogenisieren der Salzmischung;
7. Start des Besprühvorgangs, indem durch eine Zweistoffdüse Preßluft oder andere Gase sowie das Sprühmedium in das Wirbelbett eingebracht wird. Die eingebrachte Flüssigkeit löst die Salzpartikel an, so daß diese unter Ausbildung von Flüssigkeitsbrücken nach und nach aneinander kleben. Die eingebrachte Flüssigkeit wird bei diesem Vorgang eingetrocknet, wobei Feststoffbrücken entstehen.
8. Nachregelung des Heizgasvolumenstromes während des Volumen- und Gewichtszuwachses der entstehenden schwammartigen Granulate. Das Granulatwachstum entwickelt sich am besten, wenn zu Beginn viel und gegen Ende eher weniger Sprühmedium eingebracht wird. Die Temperatur muß nachgeregelt werden;
9. Drosseln des Heizgasvolumenstromes, nachdem die Granulate eine erwünschte Größe erreicht haben, um die mechanische Beanspruchung während des Nachtrocknens so gering wie möglich zu halten;
10. Entnahme der fertigen Granulate aus dem Sprühwirbelbettgranulator unter Luftabschluß, da die Granulate stark hygroskopisch sind;
11. Beispielsweise in einem evakuierbaren Trommelmischer oder einem Rotationsvakuumverdampfer wird Essigsäure in flüssiger Form bevorzugt im Vakuum in das Porensystem der Granulate eingebracht;
12. Das fertiggestellte Individualkonzentratgranulat wird unter Abschluß von Umgebungsluft in einem möglichst diffusionsfesten Beutel oder wahlweise in eine Kartusche eingefüllt.

Im folgenden wird die Versuchsreihe zur Ermittlung der Aufnahmefähigkeit des erfindungsgemäßen Granulats für Essigsäure wiedergegeben. Um festzustellen, wieviel Essigsäure von dem erfindungsgemäßen Granulat aufgenommen werden kann, wird Essigsäure mit Hilfe eines Rotavapors (Hersteller Büchi, Schweiz, Büchi B 153) beim Druck von 60 mbar (absolut) auf das Granulat aufgebracht. Danach werden die Eigenschaften des Granulates untersucht.

Bei der Messung werden 100 g des erfindungsgemäßen Granulates in ein kugelförmiges Gefäß mit einem Volumen von ca. 5 I eingebracht. Das Gefäß wird daraufhin mit Hilfe einer Vakuumpumpe auf einen Druck von 60 mbar (absolut) evakuiert. Die Essigsäure wird mit Hilfe einer Düse (0,1 mm) auf das Granulat aufgespült, während die Glaskugel rotiert.

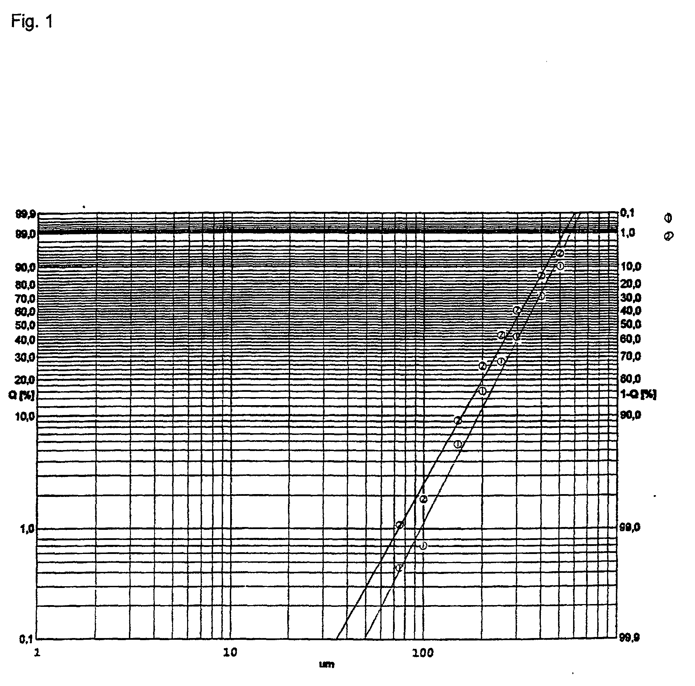

Das erfindungsgemäße Granulat wurde zunächst mit Hilfe eines Luftstrahlsiebes (Luftstrahlsieb 200 LS-N der Firma Hosokawa Alpine AG, Augsburg) auf seine Korngrößenverteilung hin untersucht. Der Schwerpunkt der Verteilung lag bei 350µm (vgl. Fig. 1). Das Granulat setzt sich zusammen aus 40% KCI, 30% MgCl₂ und 30% CaCl₂.

Mit Hilfe des Rotavapor wurden dem Granulat unterschiedliche Mengen Essigsäure zugeführt. Als Qualitätskriterium diente die freie Fließfähigkeit des Granulates mit der Essigsäure. Die Ergebnisse sind in der folgenden Tabelle 1 wiedergegeben.

| Menge Granulat [g] | Menge Essigsäure [g] | Fließfähig |
|---|---|---|
| 100 | 2 | ja |
| 100 | 4 | ja |
| 100 | 6 | ja |
| 100 | 8 | ja |
| 100 | 10 | ja |
| 100 | 12 | ja |
| 100 | 14 | ja |
| 100 | 16 | ja |
| 100 | 18 | ja |
| 100 | 20 | nein |

Aus der Tabelle 1 kann entnommen werden, daß das erfindungsgemäße Granulat in der Lage ist, bis zu 18 Gew.-% Essigsäure aufzunehmen. Bei einer Vergrößerung der Granulate ist auch eine Vergrößerung der Essigsäurekapazität zu erwarten.

Die Korngrößenverteilung des Granulats ist in der Fig. 1 wiedergegeben.

## Patentansprüche

1. Verfahren zur Herstellung eines Granulates der zur Bereitstellung einer Dialysierflüssigkeit für die Hämodialyse notwendigen Elektrolyten Kaliumchlorid (KCL), Calciumchlorid (CaCl₂) und Magnesiumchlorid (MgCl₂), dem eine gewünschte Menge Essigsäure beigemengt ist,
**dadurch gekennzeichnet,**
daß die Salze zunächst auf eine Korngröße von kleiner als 0,5 mm eingesetzt werden,
daß diese dann anschließend in trockener Gasatmosphäre auf eine Korngröße von 1-10 mm granuliert werden, wobei ein Porenvolumen im Granulat von größer als 25 Vol.% erzeugt wird und
daß darauffolgend die Essigsäure auf das Granulat aufgesprüht wird.

2. Verfahren nach Anspruch 1, dadurch gekennzeichnet, daß das Porenvolumen im Granulat zwischen 37 Vol.% und 60 Vol.% besteht.

3. Verfahren nach Anspruch 1 oder 2, dadurch gekennzeichnet, daß die drei Komponenten Kaliumchlorid, Calciumchlorid und Magnesiumchlorid getrennt voneinander zerkleinert werden.

4. Verfahren nach einem der Ansprüche 1 bis 3, dadurch gekennzeichnet, daß die zuvor zerkleinerten Salze im Wirbelbett zu größeren Granulaten unter Aufdüsen von Lösungsmittel und Abtrocknen dieses Lösungsmittels granuliert werden.

5. Verfahren nach einem der Ansprüche 1 bis 4, dadurch gekennzeichnet, daß die Lösungsmittel unter Vakuum abgetrocknet werden.

6. Verfahren nach einem der Ansprüche 1 bis 3, dadurch gekennzeichnet, daß in einem Wirbelbett ausgehend von einer Vorlage aus festen Partikeln die Granulate unter Aufdüsung von in einem Lösungsmittel aufgelösten Salzen und anschließendem Abtrocknen des Lösungsmittels granuliert werden.

7. Verfahren nach einem der Ansprüche 1 bis 3, dadurch gekennzeichnet, daß die Granulierverfahren nach den Ansprüchen 4 bis 6 in einem Wirbeibett miteinander kombiniert werden, indem mindestens eines der eingesetzten Salze als zur Vorlage dienende feste Partikel vorgelegt wird und die anderen Salze als in Lösung aufgedüste Feststoffe eingesetzt werden.

8. Verfahren nach Anspruch 7, dadurch gekennzeichnet, daß das Lösungsmittel Wasser ist.

9. Verfahren nach einem der Ansprüche 1 bis 8, dadurch gekennzeichnet, daß die End- und Resttrocknung des Granulats in einem Festbett, in einem Schachttrockner oder in einem Bandtrockner erfolgt.

10. Verfahren nach einem der Ansprüche 1 bis 9, dadurch gekennzeichnet, daß im Granulierschritt im Inneren des erzeugten Granulats feine Poren und im äußeren Bereich des Granulats grobe Poren erzeugt werden.

11. Verfahren nach einem der Ansprüche 1 bis 10, dadurch gekennzeichnet, daß das hergestellte Granulat unter Feuchtigkeitsabschluß von der Wirbelschicht zu den zur Durchführung der weiteren Verfahrensschritte vorgesehenen Apparaten unter Luftabschluß transportiert wird.

12. Verfahren nach einem der Ansprüche 1 bis 11, dadurch gekennzeichnet, daß die Essigsäure in einem mechanisch schonenden Mischer unter Vakuum auf das Granulat aufgedüst wird.

13. Verfahren nach einem der Ansprüche 1 bis 12, dadurch gekennzeichnet, daß dem Granulat zusätzlich Glucose beigemengt wird.

14. Granulat zur Bereitstellung einer Dialyseflüssigkeit mit den Elektrolyten Kaliumchlorid, Calciumchlorid und Magnesiumchlorid und flüssige Essigsäure,
dadurch gekennzeichnet,
daß das Granulat eine Korngröße von mindestens 1 mm aufweist, daß das Granulat eine Porengröße von wenigstens 25 Vol.-% besitzt und daß das Granulat eine Mindestmenge von 18 Gew.-% Essigsäure aufgenommen hat und weiterhin rieselfähig ist.

## Claims

1. Process for producing granules of the electrolytes which are needed to prepare a dialysis fluid for haemodialysis, potassium chloride (KCl), calcium chloride (CaCl₂) and magnesium chloride (MgCl₂), which granules are mixed with a desired amount of acetic acid, characterized in that the salts are initially employed in a particle size of less than 0.5 mm,
in that the latter are then granulated in a dry gas atmosphere to a particle size of 1-10 mm, producing a pore volume in the granules of more than 25% by volume, and in that the acetic acid is subsequently sprayed onto the granules.

2. Process according to Claim 1, characterized in that the pore volume in the granules comprises between 37% by volume and 60% by volume.

3. Process according to Claim 1 or 2, characterized in that the three components potassium chloride, calcium chloride and magnesium chloride are comminuted separately from one another.

4. Process according to any of Claims 1 to 3, characterized in that the previously comminuted salts are granulated in a fluidized bed to larger granules while spraying on solvent and drying off this solvent.

5. Process according to any of Claims 1 to 4, characterized in that the solvents are dried off in vacuo.

6. Process according to any of Claims 1 to 3, characterized in that the granules are granulated in a fluidized bed, starting from introduced solid particles, while spraying on salts dissolved in a solvent, and subsequently drying off the solvent.

7. Process according to any of Claims 1 to 3, characterized in that the granulation processes according to Claims 4 to 6 are combined together in one fluidized bed by initially introducing at least one of the salts employed as solid particles, and employing the other salts as solids sprayed on in solution.

8. Process according to Claim 7, characterized in that the solvent is water.

9. Process according to any of Claims 1 to 8, characterized in that the final and residual drying of the granules takes place in a fixed bed, in a tower dryer or in a belt dryer.

10. Process according to any of Claims 1 to 9, characterized in that in the granulation step there is production of fine pores inside the granules produced, and coarse pores in the outer region of the granules.

11. Process according to any of Claims 1 to 10, characterized in that the produced granules are transported with exclusion of moisture from the fluidized bed to the apparatuses intended for carrying out the further process steps, with exclusion of air.

12. Process according to any of Claims 1 to 11, characterized in that the acetic acid is sprayed onto the granules in vacuo in a mechanically gentle mixer.

13. Process according to any of Claims 1 to 12, characterized in that glucose is additionally mixed with the granules.

14. Granules for preparing a dialysis fluid having the electrolytes potassium chloride, calcium chloride and magnesium chloride and liquid acetic acid, characterized in that the granules have a particle size of at least 1 mm, in that the granules have a pore size of at least 25% by volume, and in that the granules have taken up a mimumum amount of 18% by weight of acetic acid and are still free-flowing.

## Revendications

1. Procédé de fabrication d'un granulat des électrolytes chlorure de potassium (KCL), chlorure de calcium (CaCl₂) et chlorure de magnésium (MgCl₂) requis pour la mise à disposition d'un liquide dialyseur pour l'hémodialyse, auquel est ajouté une quantité souhaitée d'acide acétique, caractérisé en ce
que les sels sont utilisés d'abord à une grandeur de grain inférieure à 0,5mm,
en ce que ceux-ci sont ensuite granulés dans une atmosphère gazeuse sèche à une grandeur de grain de 1-10mm, où est produit un volume de pores dans le granulat supérieur à 25 % en vol. et
en ce qu'ensuite, l'acide acétique est pulvérisé sur le granulat.

2. Procédé selon la revendication 1, caractérisé en ce que le volume des pores dans le granulat est compris entre 37% en vol. et 60 % en vol.

3. Procédé selon la revendication 1 ou 2, caractérisé en ce que les trois composants chlorure de potassium, chlorure de calcium et chlorure de magnésium sont fragmentés séparément les uns des autres.

4. Procédé selon l'une des revendications 1 à 3, caractérisé en ce que les sels auparavant fragmentés sont granulés dans un lit fluidisé en de plus grands granulats, par une projection de solvant et le séchage de ce solvant.

5. Procédé selon l'une des revendications 1 à 4, caractérisé en ce que les solvants sont séchés sous vide.

6. Procédé selon l'une des revendications 1 à 3, caractérisé en ce que, dans un lit fluidisé en partant d'un support de particules solides, les granulés sont granulés par une projection de sels dissous dans un solvant et un séchage subséquent du solvant.

7. Procédé selon l'une des revendications 1 à 3, caractérisé en ce que les procédés de granulation selon les revendications 4 à 6 sont combinés dans un lit fluidisé, en ce qu'au moins l'un des sels utilisés est présenté sous forme de particules solides servant de support et les autres sels sont utilisés comme matières solides pulvérisées en solution.

8. Procédé selon la revendication 7, caractérisé en ce que le solvant est de l'eau.

9. Procédé selon l'une des revendications 1 à 8, caractérisé en ce que le séchage final et résiduel du granulat a lieu dans un lit fixe, un sécheur à couloir ou dans un sécheur à bande.

10. Procédé selon l'une des revendications 1 à 9, caractérisé en ce que lors de l'étape de granulation sont produits, à l'intérieur du granulat produit, des pores fins et dans la zone extérieure du granulat des pores grossiers.

11. Procédé selon l'une des revendications 1 à 10, caractérisé en ce que le granulat produit est transporté, avec une exclusion de l'humidité, du lit fluidisé aux appareils prévus pour l'exécution des étapes de procédé suivantes, sous exclusion d'air.

12. Procédé selon l'une des revendications 1 à 11, caractérisé en ce que l'acide acétique est pulvérisé dans un mélangeur à ménagement mécanique sous vide sur le granulat.

13. Procédé selon l'une des revendications 1 à 12, caractérisé en ce qu'on ajoute au granulat en plus de la glucose.

14. Granulat pour la mise à disposition d'un liquide de dialyse avec les électrolytes chlorure de potassium, chlorure de calcium et chlorure de magnésium et d'acide acétique liquide, caractérisé
en ce que le granulat a une grandeur de grain d'au moins 1mm, que le granulat a une taille de pores d'au moins 25 % en vol. et en ce que le granulat a absorbé une quantité minimale de 18 % en poids d'acide acétique et est toujours coulant.
